# EUROPÄISCHE PATENTSCHRIFT

(11) **EP 1 617 858 B1**
(45) Veröffentlichungstag und Bekanntmachung des Hinweises auf die Patenterteilung: **03.11.2010**
(21) Anmeldenummer: 04722803.6
(22) Anmeldetag: 24.03.2004
(51) Int. Cl.: A61P 31/16, A61K 38/07, A61K 38/55

(54) **Caspase 3, 8, 9 oder 10 Inhibitoren in Kombination mit MEK Inhibitoren zur Behandlung von Influenza**
Caspase 3, 8, 9 or 10 inhibitors in combination with MEK inhibitors for the treatment of influenza
Des inhibiteurs des caspases 3, 8, 9 ou 10 en combinaison avec des inhibiteurs de la MEK pour le traitement de l'influenza

(30) Priorität: 26.03.2003 DE 10313636
(43) Veröffentlichungstag der Anmeldung: 25.01.2006
(73) Patentinhaber: Activaero GmbH, 35285 Gemünden (DE)
(72) Erfinder: LUDWIG, Stephan, 48161 Münster (DE); PLANZ, Oliver, 72108 Rottenburg (DE); SEDLACEK, Hans-Harald, 35041 Marburg (DE); PLESCHKA, Stephan, 35390 Giessen (DE)
(74) Vertreter: Vossius & Partner
(86) Internationale Anmeldenummer: PCT/DE2004/000646
(87) Internationale Veröffentlichungsnummer: WO 2004/085682

(56) Entgegenhaltungen:
- US-B1- 6 303 374
- TAKIZAWA TAKENORI ET AL: "Recruitment of apoptotic cysteine proteases (caspases) in influenza virus-induced cell death" MICROBIOLOGY AND IMMUNOLOGY, Bd. 43, Nr. 3, 1999, Seiten 245-252, XP009037647 ISSN: 0385-5600 in der Anmeldung erwähnt
- OLSEN CHRISTOPHER W ET AL: "Bcl-2 alters influenza virus yield, spread, and hemagglutinin glycosylation" JOURNAL OF VIROLOGY, Bd. 70, Nr. 1, 1996, Seiten 663-666, XP002299892 ISSN: 0022-538X
- WURZER WALTER J ET AL: "Caspase 3 activation is essential for efficient influenza virus propagation." EMBO (EUROPEAN MOLECULAR BIOLOGY ORGANIZATION) JOURNAL, Bd. 22, Nr. 11, 2. Juni 2003 (2003-06-02), Seiten 2717-2728, XP002296339 ISSN: 0261-4189
- PLESCHKA S ET AL: "INFLUENZA VIRUS PROPAGATION IS IMPAIRED BY INHIBITION OF THE RAF/MEK/ERK SIGNALLING CASCADE" NATURE CELL BIOLOGY, MACMILLAN PUBLISHERS, GB, Bd. 3, Nr. 3, März 2001 (2001-03), Seiten 301-305, XP001172412 ISSN: 1465-7392 in der Anmeldung erwähnt
- PARK JONG-WOOK ET AL: "Bcl-2 overexpression attenuates resveratrol-induced apoptosis in U937 cells by inhibition of caspase-3 activity" CARCINOGENESIS (OXFORD), Bd. 22, Nr. 10, Oktober 2001 (2001-10), Seiten 1633-1639, XP002299894 ISSN: 0143-3334
- RENVOIZÉ C ET AL: "Bcl-2 expression in target cells leads to functional inhibition of caspase-3 protease family in human NK and lymphokine-activated killer cell granule-mediated apoptosis." JOURNAL OF IMMUNOLOGY (BALTIMORE, MD. : 1950) 1 JUL 1997, Bd. 159, Nr. 1, 1. Juli 1997 (1997-07-01), Seiten 126-134, XP002299895 ISSN: 0022-1767

## Beschreibung

### Gebiet der Erfindung.

Die vorliegende Erfindung betrifft ein Kombinationspräparat gemäß dem Oberbegriff des Anspruchs 1. Hintergrund und Stand der Technik.

Infektionen mit RNA- oder DNA-Viren stellen eine bedeutende Gesundheitsbedrohung für Mensch und Tier dar. Beispielsweise gehören Infektionen mit Influenza-Viren immer noch zu den großen Seuchen der Menschheit und fordern Jahr für Jahr eine Vielzahl an Todesopfern. Sie sind gesamtwirtschaftlich, etwa durch krankheitsbedingte Arbeitsunfähigkeit, ein immenser Kostenfaktor. Von ebenfalls wichtiger wirtschaftlicher Bedeutung sind beispielsweise Infektionen mit dem Borna Disease Virus (BDV), das vor allem Pferde und Schafe befüllt, jedoch auch schon beim Menschen isoliert wurde und hier in Zusammenhang mit neurologischen Erkrankungen gebracht wird.

Das Problem der Bekämpfung von insbesondere RNA-Viren ist die durch eine hohe Fehlerrate der viralen Polymerasen verursachte Wandlungsfähigkeit der Viren, die sowohl die Herstellung geeigneter Impfstoffe als auch das Entwickeln von antiviralen Substanzen sehr schwierig macht.

Darüberhinaus hat sich gezeigt, dass die Anwendung von antiviralen Substanzen, die direkt gegen Funktionen des Virus gerichtet sind, zwar zu Therapiebeginn eine gute antivirale Wirkung zeigen, aber mutationsbedingt sehr schnell zur Selektion resistenter Varianten führt. Ein Beispiel hierfür ist das anti-Influenza-Agens Amantadin und dessen Derivate, welches bzw. welche gegen ein Transmembranprotein des Virus gerichtet ist bzw. sind. Innerhalb kurzer Zeit nach Anwendung bilden sich resistente Varianten des Virus.

Ein weiteres Beispiel sind die neuen Therapeutika für Influenzainfektionen, die das Influenza-virale Oberflächenprotein Neuraminidase hemmen. Hierzu gehört beispielsweise Relanza. In Patienten wurden bereits Relanza-resistente Varianten gefunden (Gubareva et al., J Infect Dis 178, 1257-1262, 1998). Die Hoffnungen, die in dieses Therapeutikum gelegt wurden, konnten somit nicht erfüllt werden.

Aufgrund ihrer meist sehr kleinen Genome und damit verbundener begrenzter Kodierungskapazität für replikationsnotwendige Funktionen sind alle Viren in starkem Maße auf Funktionen ihrer Wirtszellen angewiesen. Durch Einflussnahme auf solche zelluläre Funktionen, die für die virale Replikation notwendig sind, ist es möglich, die Virusreplikation in der infizierten Zelle negativ zu beeinträchtigen. Hierbei besteht für das Virus nicht die Möglichkeit, durch Anpassung, insbesondere durch Mutationen, die fehlende zelluläre Funktion zu ersetzen, um so dem Selektionsdruck zu entweichen. Dies konnte am Beispiel des Influenza A Virus mit relativ unspezifischen Hemmstoffen gegen zelluläre Kinasen und Methyltransferasen bereits gezeigt werden (Scholtissek und Müller, Arch Virol 119, 111-118, 1991).

Es ist bekannt, dass Zellen über eine Vielzahl von Signalübertragungswegen verfügen, mit Hilfe derer auf die Zelle einwirkende Signale in den Zellkern übertragen werden. Dadurch kann die Zelle auf äußere Reize reagieren und mit Zellproliferation, Zellaktivierung, Differenzierung oder kontrolliertem Zelltod antworten.

Diesen Signalübertragungswegen ist gemeinsam, dass sie mindestens eine Kinase enthalten, welche durch Phosphorylierung mindestens ein nachfolgend signalübertragendes Protein aktivieren.

Bei Betrachtung der zellulären Prozesse, die nach Virusinfektionen induziert werden, findet man, dass eine Vielzahl von DNA- und RNA-Viren in der infizierten Wirtszelle definierte Signalübertragungswege aktivieren, so beispielsweise den sogenannten Raf/MEK/ERK-Kinase-Signalübertragungsweg oder den MEKK/SEK/JNK Signalübertragungsweg. Neuere Daten zeigen, dass die Inhibition des Ras-Raf-MEK-ERK-Signalübertragungsweges durch Wirksubstanzen, welche relativ selektiv eine oder mehrere der an diesem Signalübertragungsweg beteiligten Kinasen, beispielsweise die MEK und/oder die SEK inhibieren, die intrazelluläre Vermehrung von intranukleär replizierenden Negativstrang-RNA Viren, beispielsweise von Influenza A Virus und Borna Disease Virus (BDV) drastisch hemmen kann (PCT/DE 01/01292; PCT /DE 02/02810).

Es ist bekannt, dass Viren die Apoptose der infizierten Zelle induzieren können. Dieses konnte beispielsweise für Influenza Viren in vitro und in vivo nachgewiesen werden (Fesq et al 1994; Hinshaw et al 1994; Mori et al 1995; Takizawa et al 1993). Welches Virusprotein hierbei proapoptotisch wirkt, ist noch nicht eindeutig geklärt, möglicherweise wird die Apoptose der Wirtszelle durch die Bildung von Interferon induziert (Balachandran et al 2000) oder durch proapoptotische Virusproteine wie beispielsweise PB1-F2 (Chen et al 2001).

Welchen Einfluss die virusinduzierte Apoptose auf die Virusvermehrung hat ist unklar. Teils wird angenommen, dass die Freisetzung proapoptotischer Virusproteine Lymphozyten in die Apoptose führen kann und es hierdurch zu einer reduzierten Immunabwehr gegen die Virusinfizierten Zellen und zu einer Begünstigung der Virusvermehrung kommen kann (van Campen et al 1989; Tumpey et al 2000). Andere vermuten, dass die Apoptose der Wirtszelle über die hierdurch verstärkte Phagozytose die Immunreaktion gegen das Virus erhöht (Watanabe et al 2002). Andererseits ist bekannt, dass eine Überexpression des antiapoptotisch wirkenden Bcl-2 die Virusvermehrung inhibiert (Hinshaw et al 1994; Olsen et al 1996). Im Gegensatz hierzu stehen Befunde, dass die Hemmung der Virus-induzierten Apoptose durch einen Caspase-Inhibitor keinen Einfluss auf die Synthese von Virus-Proteinen hatte (Takizawa et al 1999).

Die Apoptose einer Zelle kann außer durch Viren auch durch verschiedene andere proapoptotische Mechanismen und Proteine induziert werden. Gemeinsam ist diesen unterschiedlichen Mechanismen und Proteinen, dass sie eine proteolytische zelluläre Kaskadenreihe von Cysteinyl-Proteasen, genannt Caspasen, aktivieren. Die initial aktivierten Caspasen wie beispielsweise Caspase 8 und Caspase 9 aktivieren hierbei die Effektor- Kaskaden wie beispielsweise die Caspasen 3 und 6. Diese wiederum spalten eine Reihe von zellulären Substraten und bewirken hierdurch die Apoptose der betroffenen Zelle (Übersichten bei Cohen 1997; Thornberry und Lazebnik 1998), Das Dokument F. Takenari et al., Microbiology and Immunology, 43(3),245-252 (1999) beschreibt, dass Virus-induzierte Apoptose durch caspase 3 Aktivität vermittelt wird. Technisches Problem der Erfindung.

Der Erfindung liegt das technische Problem zu Grunde, Kombinationspraparate, welche verbesserte antivirale Wirkung zeigen, anzugeben.

Grundzüge der Erfindung sowie Ausführungsformen. zur lösung des technischen Problems lehrt die Erfindung den Gegenstand des Auspruchs 1, sowie den Gegenstand des Auspruchs 2. Es wurde gefunden, dass i) Influenzaviren für ihre Vermehrung die zellulären Caspasen, im besonderen Caspase 3 benötigen und dass in Zellen ohne Caspase 3 die Virus-Genom-Ribonukleoprotein-Komplexe nicht durch die Poren der Kernmembran in das Zytoplasma diffundieren können, sondern im Kern verbleiben, ii) die Hemmung mindestens einer zellulären Caspase, im besonderen die Hemmung der Caspase 3 zu einer deutlichen Inhibition der Vermehrung von Negativ-Strang RNA Viren, im besonderen von Influenza Viren führt und iii) die Kombination eines Inhibitors für eine Caspase, im Besonderen von Caspase 3, mit einer anderen antiviral wirkenden Substanz, nämlich mit einem Inhibitor für eine zelluläre Kinase, einen synergistischen Effekt auf die Hemmung der Virusvermehrung aufweist.

Die Wirkung von Caspase-Inhibitoren auf die Vermehrung von Viren, im besonderen von Negativ-Strang-RNA Viren, beispielsweise von Influenzaviren wird dadurch verdeutricht, das diese Inhibition der Virusvermehrung durch Hemmung der Caspase nicht verbunden ist mit einer Hemmung der Synthese von frühen und späten Virusproteinen (zum Beispiel NP oder NS1 (früh) noch von Matrix-Proteinen (M1, spät)) und immer noch beobachtet wurde, wenn der Caspase Inhibitor erst 4h nach Infektion zugegeben wurde.

Die Verabreichung des Kombinationspräparates kann als Mischung der Wirksubstanzen erfolgen. Die Wirksubstanzen können pro Verabreichung jedoch auch getrennt voneinander an dem gleichen Ort, beispielsweise intravenös, oder auch an voneinander getrennten Orten, gleichzeitig oder auch zu unterschiedlichen Zeitpunkten innerhalb eines Zeitraumes, in welchem die zuerst verabreichte Substanz noch Wirkung zeigt, beispielsweise in einem Zeitraum von drei Tagen, verabreicht werden.

Vorzugweise werden die Wirksubstanzen gemäß vorliegender Erfindung für die lokale oder systemische Verabreichung in einen Organismus mit Hilfe der dem Fachmann geläufigen Methoden und Hilfs- und/oder Zusatzstoffen zu einem Arzneimittel zubereitet.

Geeignete Hilfs- und Zusatzstoffe, die beispielsweise der Stabilisierung oder Konservierung des Arzneimittels oder Diagnostikums dienen, sind dem Fachmann allgemein bekannt (siehe z. B. Sucker H et al. (1991) Pharmazeutische Technologie, 2. Auflage, Georg Thieme Verlag, Stuttgart). Beispiele für solche Hilfs- und/oder Zusatzstoffe sind physiologische Kochsalzlösungen, Ringer-Dextrose, Dextrose, Ringer-Laktat, entmineralisiertes Wasser, Stabilisatoren, Antioxidantien, Komplexbildner, antimikrobielle Verbindungen, Proteinaseinhibitoren und/oder inerte Gase.

Die lokale Verabreichung kann beispielsweise auf die Haut, auf die Schleimhaut, in eine Körperhöhle, in ein Organ, in ein Gelenk oder in das Binde- oder Stützgewebe, durch nasale Verabreichung oder durch Inhalation erfolgen. Die systemische Verabreichung erfolgt vorzugsweise in den Blutkreislauf, in die Peritonealhöhle oder in die Bauchhöhle.

Die Arzneimittelzubereitung enthaltend die erfindungsgemäßen Wirksubstanzen richtet sich nach der Art der Wirksubstanzen und der Art ihrer Verabreichung und kann beispielsweise eine Lösung, eine Suspension, eine Salbe, ein Puder, eine Sprayform oder eine andere Inhalationszubereitung darstellen. Vorzugsweise werden Nukleotidsequenzen mit dem Fachmann bekannten Methoden in einen viralen Vektor oder ein Plasmid eingefügt und mit Hilfsstoffen für die Zelltransfektion versetzt. Zu diesen Hilfsstoffen gehören beispielsweise kationische Polymere oder kationische Lipide. Antisense-Oligonukleotide werden mit den dem Fachmann geläufigen Methoden derivatisiert, um sie vor dem enzymatischen Abbau durch DNAsen oder RNAsen zu schützen.

Die Wirksubstanzen gemäß der Erfindung können in Form eines Salzes, Esters, Amids oder als eine Vorstufe vorliegen, wobei vorzugsweise nur Modifikationen der Wirksubstanz verwendet werden, die keine übermäßige Toxizität, Irritationen oder allergische Reaktionen am Patienten auslösen.

Die Wirksubstanzen werden unter sterilen Bedingungen, mit einem physiologisch akzeptablen Trägerstoff und möglichen Preservativen, Puffern oder Treibmitteln, je nach Bedarf, vermischt. Derartige Trägerstoffe für Arzneimittelzubereitungen sind dem Fachmann geläufig.

Bevorzugt werden die erfindungsgemäßen Wirksubstanzen in einer einmaligen Dosis, besonders bevorzugt in mehreren Dosen verabreicht, wobei die einzelnen Dosen die maximal tolerable Dosis (MTD) der jeweiligen Wirksubstanz für den Menschen nicht übersteigt. Vorzugsweise wird eine Dosis gewählt, welche die Hälfte der MTD beträgt

Gemäß der vorliegenden Erfindung kann die Verabreichung entweder lokal oder systemisch, nur an einem Tag oder über mehrere Tage täglich oder an jedem zweiten oder dritten Tag über mehrere Wochen erfolgen, bis eine therapeutische Wirkung sichtbar wird.

Im folgenden wird die Erfindung anhand von lediglich Ausführungsformen darstellenden Beispielen näher erläutert.

### Beispiel 1 (Vergleich): Virusvermehrung in Wildtyp und in Caspase 3 defizienten Zellen

Um zu analysieren, ob Caspasen, speziell Caspase 3, eine wichtige Rolle in der Influenza Virus Vermehrung spielt, wurde die Aktivität und Expression der Protease(n) auf vier verschiedene Arten gehemmt: a) durch Zugabe eines zellpermeablen Inhibitors (Z-DEVD-FMK), welcher neben anderen Caspasen bevorzugt Caspase 3 Aktivität hemmt, b) durch Expression eines inhibitorischen Proteins von Caspasen, XIAP (X-linked inhibitor of apoptosis) (Devereaux et al Nature, 388, 300-304, 1997), welches unter anderem Caspase 3 hemmt, c) durch stabile Transfektion eines Vektors, welcher eine siRNA gegen die mRNA von Caspase 3 bildet, d) durch Untersuchung einer Zelllinie (MCF-7), welche Caspase 3-defizient ist (Jänicke et al. J Biol Chem, 273, 9357-9360) und die durch transiente Transfektion mit Procaspase 3 komplementiert wurde.

Zu a) wurde wie folgt verfahren. MDCK Zellen wurden mit dem Influenza A Virusstamm Bratislava/79 (Fowl Plague virus, FPV) mit einer Multiplizität der Infektion von 1 (M.O.I.=1) infiziert in Abwesenheit und Anwesenheit steigender Mengen des Caspase Inhibitors Z-DEVD-FMK (2, 4, 20, 40 µM, Alexis Biochemicals), welcher bevorzugt Caspase 3 hemmt. Die der höchsten Inhibitormenge entsprechende Konzentration an DMSO (2%) diente als Lösungsmittelkontrolle. Als weitere Kontrolle wurde das inaktive Inhibitor-Analogon Z-FA-FMK in einer Konzentration von 40 µM angewendet. Nach 24h wurden die Zellüberstände hinsichtlich der Menge an neu gebildeten Viren mit üblichen Methoden (Plaque Titration) untersucht. Parallel dazu wurde die Wirkung des Caspase Inhibitors durch das Maß der Spaltung des zellulären Caspase Substrates PARP (Poly-ADP-Ribose Polymerase), welches unter anderm durch Caspase 3 gespalten wird (Tewari et al Cell 81, 801-809, 1995), im Westen Blot von Zellysaten analysiert. Ebenfalls untersucht wurde die Wirkung des Inhibitors auf Expression früher und später viraler' Proteine (NS1, NP, M1) im Western Blot. In einer Variation des Experiments wurde der Inhibitor DEVD-FMK in einer Konzentration von 40 µM zugegeben und bereits nach 2h nach Infektion weggewaschen und durch frisches Medium ersetzt, bzw. erst 4h nach Infektion zugegeben. In einer weiteren Abwandlung des Experiments wurde der Breitband-Caspase Inhibitor Z-VAD-FMK vergleichend in analogen Konzentrationen sowohl in AS49, MDCK als auch in Vero Zellen eingesetzt.

Zu b) wurde wie folgt verfahren. MDCK Zellen wurden transfiziert mit einem Vektorplasmid oder mit Plasmiden welche XIAP oder Procaspase 3 exprimieren. Die Transfektion wurde mit dem Transfektionsreagenz Lipofectamine 2000 (Life Technologies) nach Standardmethoden (Ludwig et al. J Biol Chem 276,10990-10998,2001) durchgeführt. Die Transfektionseffizienzen lagen bei ca. 60%. 24h nach Transfektion erfolgte Infektion mit dem Influenza A Virusstamm Fowl Plague Virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft. Die erfolgreiche Expression der transient exprimierten Proteine wurde im Western Blot verifiziert.

Zu c) wurde wie folgt verfahren. Die Lungenepithelzelllinie A549 wurde nach Standardmethoden (Lipofectamine 2000 (Life Technologies); Ludwig et al. J Biol Chem 276,10990-10998,2001 ) mit dem Vektor pSUPER transfiziert, welcher zur Bildung kleiner interferierender dsRNA Fragmente in der Zelle (siRNA) führt (Brummelkamp et al. Science, 296, 550-553, 2002). Als Inserts dienten die folgenden Zielsequenzen der Caspase 3 (Genbank Accession No. NM004346): TGACATCTCGGTCTGGTAC (nt 417-435), CTGGACTGTGGCATTGAGA (734-755) and TACCAGTGGAGGCCGACTT (795-813) (Klone #113, #252 and #311). In einem weiteren Klon (#313) wurde eine Insertion identifiziert. Somit diente dieser Klon als Negativkontrolle. Die Konstrukte wurden gemeinsam mit dem Vektor pCAGI-puro transfiziert um die Zellen selektionierbar mit dem Antibiotikum Puromycin zu machen. 24h nach Transfektion wurden die Zellen gewaschen und daraufhin mit Medium, welches 1 µg/ml Puromycin enthielt, inkubiert. 24h später wurden die Zellen intensiv mit PBS gewaschen und neues Antibiotika-haltiges Medium zugegeben. Diese Prozedur wurde dann 7 Tage lang in Anwesenheit von 0.6 µg/ml Puromycin wiederholt. Nach 7 Tagen wurden die verschiedenen Zellen auf Expression der Caspase 3 hin untersucht. Ebenfalls nach 7 Tagen erfolgte Infektion der verschiedenen Zelllinien mit dem Influenza A Virusstamm Fowl Plague Virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft.

Zu d) wurde wie folgt verfahren. Die Caspase 3-defiziente Brustkarzinom Zelllinie MCF-7 wurden transfiziert mit einem Vektorplasmid oder einem Plasmiden welches Procaspase 3 exprimiert. Die Transfektion wurde mit dem Transfektionsreagenz Lipofectamine 2000 (Life Technologies) nach Standardmethoden (Ludwig et al. J Biol Chem 276,10990-10998,2001) durchgeführt. Die Transfektionseffizienzen lagen bei ca. 50%. 24h nach Transfektion erfolgte Infektion mit dem Influenza A Virusstamm Fowl Plague Virus (FPV) mit einer Multiplizität der Infektion von 1 (M.O.I.=1). Weitere 24h nach Infektion wurden die Titer der neu gebildeten Viren im Zellkulturüberstand in Standard Plaque-Assays auf MDCK Zellen überprüft. Die erfolgreiche Expression von Procaspase 3 wurde im Western Blot verifiziert.

Es wurden die folgenden Ergebnisse erhalten.

Zu a): der Caspase 3 Inhibitor Z-DEVD-FMK führte Dosisabhängig zur Reduktion der Influenza Virus Titer nach 24h bis etwa 60% Hemmung bei einer Konzentration von 40µM. Diese Hemmung korrelierte exakt mit der erreichten Caspase Hemmung, gemessen an der Spaltung des Caspase Substrates PARP. Dies zeigt, dass das Level der Aktivität zellulärer Caspasen, besonders von Caspase 3 direkt mit der Effizienz der Virusvermehrung korreliert und Caspase Inhibitoren zur Hemmung der Influenza Vermehrung eingesetzt werden können. Die gleiche Effizienz der Hemmung der Virusvermehrung wurde mit einem pan-Caspase Inhibitor, Z-VAD-FMK sowohl in A549 Zellen als auch in MDCK und Vero Zellen erreicht, während ein inaktives Inhibitor Analogon Z-FA-FMK keine Wirkung zeigte. Zusätzlich wurde beobachtet, dass trotz der hemmenden Effekte auf die Virusvermehrung, Z-DEVD-FMK keinen signifikanten Einfluss auf die Virusproteinexpression hatte. Dies ist ein Beleg dafür, dass Caspase Aktivität spät in der Virusreplikation benötigt wird. Dies wird unterstützt durch den Befund, dass der Inhibitor immer noch wirksam die Virusvermehrung hemmte, wenn er erst 4h nach Infektion, also zu späten Zeitpunkten des Replikationszyklus zugegeben wird. Anwesenheit von Z-DEVD-FMK in den ersten beiden Stunden nach Infektion hatte bei nachfolgender Entfernung keine signifikanten Effekte.

Zu b): Expression des Caspase-inhibitorischen Proteins XIAP führte zu einer Reduktion der Influenza Virus Titer nach 24h von etwa 50%. Diese Reduktion korrelierte weitestgehend mit der erzielten Hemmung der Caspase Aktivität, gemessen an der verminderten Spaltung des Proteins PARP, welche ebenfalls bei 40-50% der Effizienz der Ausgangsaktivität lag. Umgekehrt konnte durch Expression der Procaspase-3 eine gesteigerte Virusvermehrung in den transfizierten Zellen beobachtet werden. Dies belegt einmal mehr, dass das Level der Caspase 3 Aktivität in Zellen direkt mit der Effizienz der Influenza Virus Replikation korreliert.

Zu c): Western Blot Experimente zeigten, dass stabile Expression verschiedener siRNA Segmente in A549 Zellen zu einer mehr oder weniger starken Reduktion der Proteinlevel von Caspase 3 in den verschiedenen Zelllinien führte, während Expression eines Kontroll-siRNA Segmentes keine Wirkung zeigte. Gemäß dem Grad der Reduktion der Proteinmenge ergaben sich abgestufte Effekte auf die Influenza Virus Replikation in den verschiedenen Linien, wobei das am stärksten hemmende siRNA Segment (#113) zu einer etwa 10-fachen Reduktion der Virustiter führte. Das Kontroll-siRNA Segment (#313) hatte entsprechend keinerlei Effekt auf die Virusvermehrung. Erwähnenswert ist hier, dass die starke Expressionshemmung von Caspase 3 zu einer erhöhten Expression und Aktivität der Caspase 7 in der Zelllinie #113 führte. Dieser als Kompensationsreaktion der Zelle zu verstehende Effekt konnte die Defekte in der Virusvermehrung jedoch nicht aufheben.

Zu d): Infektion von Wildtyp oder Vektor-transfizierten MCF-7 Zellen führte nur zu sehr geringen Titern an Nachkommenviren, was anzeigt, dass die Replikationseffizienz von Influenza A Viren in diesen Caspase 3-defizienten Zellen sehr gering ist. Wurde jedoch Procaspase 3 durch transiente Transfektion in diese Zellen eingebracht, beobachtete man eine 30-fache Erhöhung der Titer von Nachkommenviren, ein weitere Beleg für die Wichtigkeit von Caspasen, besonders von Caspase 3 für eine effiziente Influenza Virus Vermehrung.

Zusammenfassend kann man aus diesen Ergebnissen den Schluss ziehen, dass der Grad der Expression und Aktivität von Caspasen, besonders von Caspase 3 direkt mit der Effizienz der Influenza Virus Replikation korreliert. Entprechend bilden Caspasen, insbesondere Caspase 3, Zielpunkte für eine anti-Influenza Virus Prophylaxe oder Therapie.

### Beispiel 2 (Vergleich): Mechanismus der Inhibition der Virusvermehrung durch einen Caspaseinhibitor.

Western Blot Analysen von Zelllysaten Caspase Inhibitorbehandelter Influenza Virus-infizierter Zellen belegten, dass trotz effizienter Hemmung der Virusvermehrung, kein Effekt auf die virale Proteinsynthese eintrat und somit ein später Schritt des Replikationszyklus, wenn die virale Proteinsynthese weitestgehend abgeschlossen ist, von Caspase Aktivität betroffen zu sein scheint (siehe auch Ergebnisse zu a)). Dies wird durch Befunde unterstützt, die zeigen, dass Caspase Inhibitoren immer noch eine effiziente Hemmung der Virusvermehrung bewirken, wenn sie erst 4h nach Infektion, also spät im Infektionszyklus, zugegeben werden, während Anwesenheit der Substanzen in den ersten zwei Stunden der Infektion bei nachfolgender Entfernung keine Wirkung hatte. Ein essentieller Schritt spät im Infektionszyklus von Influenza Viren ist der Export neu gebildeter viraler RNA in Form von Ribonukleoproteinkomplexen (RNPs) aus dem Zellkern der infizierten Zelle. Kürzlich wurde gezeigt, dass Caspase Aktivität in Zellen zu einer Erweiterung der Kernporen führt, was großen Proteinen oder Proteinkomplexen eine freie Diffusion zwischen Zellkern und Zytoplasma erlaubt (Falerio und Lazebnik J Cell Biol, 151, 951-959, 2000). Um zu analysieren, ob Caspase Aktivität regulierend in den Export von viralen Proteinen oder RNPs aus dem Zellkern eingreift und ob dies durch freie Diffusion der Proteine geschieht wurden folgende experimentelle Ansätze durchgeführt: a) Wildtyp A549 Zellen und A549 Zellen, welche eine Caspase 3 siRNA tragen wurden infiziert und in Immunfluoreszenzanalysen auf die Lokalisation der RNPs hin untersucht, b) Wildtyp MDCK Zellen wurden mit dem Caspase 3 Inhibitor Z-DEVD-FMK behandelt und infiziert und ebenfalls in Immunfluoreszenzanalysen auf die Lokalisation der RNPs hin untersucht, c) MDCK Zellen wurden mit einem Plasmid für das Influenza A Virus Nukleoprotein (NP) transfiziert und die Lokalisation des NP wurde in Immunfluoreszenzanalysen nach Apoptoseinduktion mit Staurosporin in An- und Abwesenheit eines Caspase Inhibitors untersucht, d) MDCK Zellen wurden zur Rekonstitution von RNP Komplexen mit Plasmiden transfiziert, die für das NP und die Influenza Polymerasen PB2, PB1 und PA kodieren, sowie mit einem Plasmid, welches eine Influenza Virus spezifische RNA Matrize exprimiert. Effekte auf die Lokalisation der RNP Komplexe wurde in An- und Abwesenheit von Caspase Inhibitoren in Immunfluoreszenzanalysen untersucht.

Zu a) wurde wie folgt verfahren. A549 Zellen oder A549 Zelllinen, welche siRNA Segment #113 trugen, wurden infiziert mit dem Influenza A Virusstamm Fowl Plague Virus (FPV) mit einer Multiplizität der Infektion von 3 (M.O.I.=3). 5h nach Infektion wurden die Zellen nach gängigen Methoden (Pleschka et al Nat Cell Biol 3, 301-305, 2001) der Immunfluoreszenzanalyse mit einem Ziegen anti-NP Antiserum (Robert Webster, Memphis, USA) und einem anti-Ziege Texas-Red-IgG Sekundärantikörper (Dianova) zugeführt. Die Zellkerne wurden mit DAPI gefärbt. Die Visualisierung erfolgte mit einem inversen Fluoreszenzmikroskop in einer Vergrößerung von 40x.

Zu b) wurde wie folgt verfahren. MDCK Zellen wurden infiziert mit dem Influenza A Virusstamm Fowl Plague Virus (FPV) mit einer Multiplizität der Infektion von 5 (M.O.I.=5) in Anwesenheit von DMSO (2%), dem Caspase 3 Inhibitor Z-DEVD-FMK (40 µM, Alexis Biochemicals), dem inaktiven Inhibitor Analogon Z-FA-FMK (40 µM, Alexis Biochemicals) oder dem MEK Inhibitor U0126 (50 µM, Taros Coustom Biochemicals). 5 Stunden nach Infektion wurden die Zellen nach gängigen Methoden (Pleschka et al Nat Cell Biol 3, 301-305, 2001) der Immunfluoreszenzanalyse mit einem Ziegen anti-NP Antiserum (Robert Webster, Memphis, USA) und einem anti-Ziege Texas-Red-IgG Sekundärantikörper (Dianova) zugeführt. Die Zellkerne wurden mit DAPI gefärbt, Färbung des Zytoskeletts erfolgte mit Phalloidin-FITC. Die Visualisierung erfolgte mit einem inversen Fluoreszenzmikroskop in einer Vergrößerung von 40x.

Zu c) wurde wie folgt verfahren. MDCK Zellen wurden transfiziert mit Plasmiden, welche für die Influenza A Virus Proteine PB2, PB1, PA und NP kodieren, sowie mit einem Plasmid welches eine antisense-RNA für das Grünfluoreszierende Protein flankiert von Influenza-Virus spezifischen Promotorelementen als Matrize für den Polymerasekomplex bildet. Es ist bekannt, dass Expression dieser Plasmide zu einer Rekonstitution der RNP Komplexe führt, was durch Expression des Reportergens, hier GFP, angezeigt wird (Pleschka et al J Virol, 70, 4188-4192, 1996). Die Transfektion wurde mit dem Transfektionsreagenz Lipofectamine 2000 (Life Technologies) nach Standardmethoden (Ludwig et al. J Biol Chem 276,10990-10998,2001) durchgeführt. 16 Stunden nach Transfektion wurden die Zellen für 5h mit DMSO, Staurosporin (1M) und DMSO, Staurosporin (1M) und Z-DEVD-FMK (40 µM), oder Staurosporin (1M) und Leptomycin B (2ng/ml) behandelt. Danach wurden die Zellen nach gängigen Methoden (Pleschka et al Nat Cell Biol 3, 301-305, 2001) der Immunfluoreszenzanalyse mit einem Ziegen anti-NP Antiserum (Robert Webster, Memphis, USA) und einem anti-Ziege Texas-Red-IgG Sekundärantikörper (Dianova) zugeführt. Die Zellkerne wurden mit DAPI gefärbt, Die Visualisierung erfolgte mit einem inversen Fluoreszenzmikroskop in einer Vergrößerung von 40x.

Zu d) wurde wie folgt verfahren. MDCK Zellen wurden transfiziert mit einem Plasmid welches für das Influenza A Virus NP kodiert. Die Transfektion wurde mit dem Transfektionsreagenz Lipofectamine 2000 (Life Technologies) nach Standardmethoden (Ludwig et al. J Biol Chem 276,10990-10998,2001) durchgeführt. 16 Stunden nach Transfektion wurden die Zellen für 5h mit DMSO, Staurosporin (1M) und DMSO, Staurosporin (1M) und Z-DEVD-FMK (40 µM), oder Staurosporin (1M) und U0126 (40 µM) behandelt. Danach wurden die Zellen nach gängigen Methoden (Pleschka et al Nat Cell Biol 3, 301-305, 2001) der Immunfluoreszenzanalyse mit einem Ziegen anti-NP Antiserum (Robert Webster, Memphis, USA) und einem anti-Ziege Texas-Red-IgG Sekundärantikörper (Dianova) zugeführt. Die Zellkerne wurden mit DAPI gefärbt, Die Visualisierung erfolgte mit einem inversen Fluoreszenzmikroskop in einer Vergrößerung von 40x.

Es wurde die folgenden Ergebnisseerhalten.

Zu a): Vergleich der Lokalisation der RNP Komplexe (gemäß der Detektion mit einem Antiserum gegen das virale Nukleoprotein (NP), dem Hauptbestandteil der RNPs, zeigte, dass in infizierten A549 Zellen welche siRNA-abhängig eine stark reduzierte Expression von Caspase 3 aufweisen, die RNPs 5h nach Infektion im Zellkern zurückgehalten werden, während in Wildtyp A549 Zellen zur gleichen Zeit die RNPs bereits effizient im Zytoplasma akkumulieren. Dies zeigt, dass der Grad der Caspase 3 Expression mit der Wanderungseffizienz der RNPs aus dem Zellkern korreliert und deutet an, dass dieser Effekt Caspase 3 vermittelt ist.

Zu b): Vergleich der Lokalisation der RNP Komplexe in infizierten MDCK Zellen, welche mit Lösungsmittel oder verschiedenen Inhibitoren inkubiert wurden, zeigte dass die Wanderung der RNPs aus dem Zellkern 5h nach Infektion effizient sowohl durch den Caspase Inhibitor Z-DEVD als auch durch den MEK Inhibitor U0126 gehemmt werden kann, nicht jedoch durch das inaktive Caspase Inhibitor Analogon Z-FA-FMK. Dies ist ein weiterer Beleg, dass Caspasen, insbesondere Caspase 3 den effizienten Export von RNPs aus dem Zellkern vermitteln.

Zu c): Nach transienter Expression in unstimulierten Zellen zeigte das Influenza Virus NP eine nukleäre Lokalisation. Wurde jedoch in diesen Zellen Caspase Aktivität durch Zugabe des Apoptose Induktors Staurosporin induziert, fand man das Nukleoprotein über die ganze Zelle verteilt. Dieses "Ausbluten" aus dem Zellkern konnte verhindert werden durch Zugabe des Caspase 3 Inhibitors Z-DEVD, nicht jedoch durch einen Inhibitor des aktiven Kernexports, Leptomycin B. Dies zeigt an, dass Caspase Aktivität die freie Diffusion von großen Proteinen vermutlich durch ein proteolytisches Erweitern der Kernporen vermittelt und somit das Wandern des NP ins Cytoplasma fördert.

Zu d): Nach transienter Expression der viralen Proteine PB2, PB1, PA und NP in MDCK Zellen, welche zusätzlich ausgehend von einem Plasmid eine RNA Matrize mit Influenza-Virus spezifischen Promotorregionen exprimieren, die ein GFP Reportergen flankieren, fand man grün-gefärbte Zellen in der Kulturschale, was anzeigt, dass in diesen Zellen intakte RNP Komplexe gebildet wurden. Sowohl GFP als auch die RNP Komplexe befanden sich in unstimulierten Zellen im Nukleus. Wurde jedoch in diesen Zellen Caspase Aktivität durch Zugabe des Apoptose Induktors Staurosporin induziert, fand man sowohl das GFP als auch die RNP Komplexe wiederum über die ganze Zelle verteilt. Entsprechend konnte dieses "Ausbluten" aus dem Zellkern verhindert werden durch Zugabe des Caspase 3 Inhibitors Z-DEVD, nicht jedoch durch einen Inhibitor des aktiven Kernexports, U0126. Dies zeigt an, dass Caspase Aktivität die freie Diffusion von sehr großen Proteinkomplexen vermutlich durch ein proteolytisches Erweitern der Kernporen vermittelt und somit das Wandern der RNPs ins Zytoplasma fördern. Interessant ist hierbei auch, dass die betreffenden Zellen, belegt durch die Hemmbarkeit mit Z-DEVD-FMK, zwar Caspase Aktivität aufweisen, jedoch ansonsten keine morphologischen Zeichen apoptotischer Zellen, wie beispielsweise Membranausstülpungen oder kondensierte Kerne tragen. Dies zeigt, dass bereits initiale Ereignisse der Apoptoseinduktion, wie frühe Caspase Aktivierung ausreichen, um den besseren Kernexport der Proteinkomplexe zu vermitteln. Volle Exekution des apoptotischen Programms ist nicht nötig oder wäre gar kontraproduktiv.

### Beispiel 3 (erfindungsgemäß): synergistische Wirkung eines Caspaseinhibitors und eines Kinaseinhibitors in der Hemmung der Virusvermehrung

Es ist bekannt, dass der Export von Influenza Virus RNPs zumindest teilweise durch aktiven Kernexport vermittelt wird (O'Neill et al EMBO J, 17, 288-296, 1998) und entsprechend mit Hemmstoffen der aktiven Kernexportmaschinerie wie Leptomycin B gehemmt werden kann. Es ist ebenfalls bekannt dass der RNP Export in späten Phasen der Replikation durch Hemmung der Raf/MEK/ERK Kinäse-Kaskade, unter anderem durch den MEK Inhibitor U0126 gehemmt werden kann, wobei es sich hier um das Interferieren mit einem aktiven Exportmechanismus handelt. Überraschend wurde im Rahmen der Erfindung gefunden, dass der Kernexport von Influenza Virus RNPs alternativ auch durch Caspase Inhibitoren gehemmt werden kann, wobei es sich hier weitestgehend um das Blockieren eines passiven Prozesses handelt. Es sollte nun geklärt wären ob sich a) der Caspaseaktivierende Signalweg und die Raf/MEK/ERK Kaskade gegenseitig beeinflussen und b) ob durch Hemmung des aktiven Exportes durch U0126 und gleichzeitiger Hemmung der verstärkten passiven Diffusion durch Caspase Inhibitoren, also gewissermassen Blockierung zweier alternativer Exportmechanismen, ein synergistischer Hemmungseffekt auf die Influenza Virusreplikation erzielt werden kann.

Zu a) wurde wie folgt verfahren. A549 Zellen wurden infiziert mit dem Influenza A Virusstamm Fowl Plague Virus (FPV) mit einer Multiplizität der Infektion von 1 (M.O.I.=1) in Anwesenheit von DMSO (2%), dem Caspase 3 Inhibitor Z-DEVD-FMK (40 µM, Alexis Biochemicals), oder dem MEK inhibitor U0126 (40 µM, Taros Coustom Biochemicals). 24 Stunden nach Infektion wurden die Zellen lysiert und die Lysate sowohl einem anti-PARP Western Blot zur Bestimmung der Caspase Aktivität als auch einem ERK Immunkomplexkinaseassay nach gängigen Methoden ((Pleschka et al Nat Cell Biol 3, 301-305, 2001) zur Bestimmung der Aktivität des Raf/MEK/ERK Signalwegs in den infizierten und behandelten Zellen zugeführt.

Zu b) wurde wie folgt verfahren. A549 Zellen und Caspase 3-defiziente MCF-7 Zellen wurden infiziert mit dem Influenza A Virusstamm Fowl Plague Virus (FPV) mit einer Multiplizität der Infektion von 1 (M.O.I.=1) in Anwesenheit von DMSO (2%), dem Caspase 3 Inhibitor Z-DEVD-FMK (40 µM, Alexis Biochemicals), oder dem MEK Inhibitor U0126 (40 µM, Taros Coustom Biochemicals). 9h und 24h nach Infektion wurden die Titer der neu gebildeten Viren im Zellkulturüberstand in Standard Plaque-Assays auf MDCK Zellen überprüft.

Es wurden die folgenden Ergebnisse erhalten.

Zu a): Hemmung von Caspase 3 in inifizierten Zellen führte zu einer verminderten Spaltung des Caspase Substrats PARP, nicht jedoch zu einer verminderten Aktivierung des Raf/MEK/ERK Signalweges, gemessen am Grad der Virus-induzierten Aktivität von ERK im Immunkomplexkinaseassay, welche weitestgehend identisch in DMSO und Z-DEVD-FMK behandelten Zellen war. Ebenso führte Hemmung von MEK durch U0126 in Konzentrationen welche effizient die Virus-induzierte Aktivierung von ERK hemmten, nicht zu einer veränderten Spaltung von PARP. Dies deutet an, dass die Caspase 3 abhängige Kaskade und der Raf/MEK/ERK Signalweg unabhängig voneinander verschiedene Prozesse vermitteln die alternativ den RNP Export fördern und damit die Virusvermehrung effizienter gestalten.

Zu b): wurden in A549 Zellen gleichzeitig Caspasen, bevorzugt Caspase 3, durch Z-DEVD-FMK sowie die Raf/MEK/ERK Kaskade gehemmt, konnte man einen synergistischen Hemmeffekt auf die Virusreplikation sowohl nach 9h als auch nach 24h beobachten. Damit konnte also die suboptimale Hemmwirkung von weniger als einer Zehnerpotenz durch die isoliert angewandten Agenzien bis >1 log Stufe durch Kombinationsgabe verstärkt werden. In Caspase 3-defizienten MCF-7 Zellen hatte Z-DEVD-FMK wie erwartet keine Wirkung auf die Virusvermehrung, Allerdings wurden die bereits geringen Titer von Nachkommenviren aus diesen Zellen nochmals durch Anwendung von U0126 gesenkt. Diese Befunde zeigen, dass in der Tat die Caspase Kaskade und der Raf/MEK/ERK Signalweg zwei alternative Prozesse zur effizienten Unterstützung der Virusvermehrung regulieren und das gemäß dieser Befunde kombinatorische Anwendung von Caspase Inhibitoren und von MEK Inhibitoren ideal geeignet ist um effizient die Vermehrung von Influenza Viren zu hemmen.

## Patentansprüche

1. Kombinationspräparat zur verwendung zur Therapie einer Viruserkrankung, hervorgerufen durch Influenza Viren, enthaltend mindestens eine Wirksubstanz, ausgewählt aus der Gruppe bestehend aus Inhibitoren der Caspase 3, nämlich Z-DEVD-FMK, Ac-DEVD-CHO, Ac-DMQD-CHO, Z-D(OMe)E(OMe)VD(OMe)-FMK, Z-D(OMe)QMD(OMe)-FMK, und Inhibitoren von zellulären Caspasen, welche Caspase 3 aktivieren können, nämlich die Caspase 8 Inhibitoren Z-LE(OMe)TD(OMe)-FMK, Ac-ESMD-CHO, Ac-IETD-CHO, Z-IETD-FMK, die Caspase 9 Inhibitoren Z-LE(OMe)HD(OMe)-FMK, Z-LEHD-FMK, Ac-LEHD-CHO, und die Caspase 10 Inhibitoren Ac-AEVD-CHO, Z-AEVD-FMK, die eine zelluläre Caspase derart hemmt, dass eine Virusvermehrung gehemmt wird,
**dadurch gekennzeichnet,**
**dass** zusätzlich mindestens eine antiviral wirkende Substanz, ausgewählt aus der Gruppe bestehend aus den MEK Inhibitoren 2-(2-Amino-3-methoxyphenyl)-4-oxo-4H-(1)benzopyran, U0126, PD18453, PD98059,
enthalten ist.

2. Verwendung zumindest eines Caspase Inhibitors, ausgewählt aus der Gruppe bestehend aus Inhibitoren der Caspase 3, nämlich Z-DEVD-FMK, Ac-DEVD-CHO, Ac-DMQD-CHO, Z-D(OMe)E(OMe)VD(OMe)-FMK, Z-D(OMe)QMD(OMe)-FMK, und Inhibitoren von zellulären Caspasen, welche Caspase 3 aktivieren können, nämlich die Caspase 8 Inhibitoren Z-LE(OMe)TD(OMe)-FMK, Ac-ESMD-CHO, Ac-IETD-CHO, Z-IETD-FMK, die Caspase 9 Inhibitoren Z-LE(OMe)HD(OMe)-FMK, Z-LEHD-FMK, Ac-LEHD-CHO, und die Caspase 10 Inhibitoren Ac-AEVD-CHO, Z-AEVD-FMK und eines Inhibitors einer oder mehrerer zellulärer Kinasen, ausgewählt aus der Gruppe bestehend aus den MEK Inhibitoren 2-(2-Amino-3-methoxyphenyl)-4-oxo-9H-(1)benzopyran, U0126, PD18453, PD98059 zur Herstellung eines Kombinationspräparates nach Anspruch 1 zur Behandlung einer Influenza Infektion.

## Claims

1. A combination preparation for use in the therapy of a virus disease caused by influenza viruses, containing at least one active substance, selected from the group consisting of caspase 3 inhibitors, i.e. Z-DEVD-FMK, Ac-DEVD-CHO, Ac-DMQD-CHO, Z-D(OMe)E(OMe)VD(OMe)-FMK, Z-D(OMe)QMD(OMe)-FMK, and inhibitors of cellular caspases which are capable of activating caspase 3, i.e. the caspase 8 inhibitors Z-LE(OMe)TD(OMe)-FMK, Ac-ESMD-CHO, Ac-IETD-CHO, Z-IETD-FMK, the caspase 9 inhibitors Z-LE(OMe)HD(OMe)-FMK, Z-LEHD-FMK, Ac-LEHD-CHO, and the caspase 10 inhibitors Ac-AEVD-CHO, Z-AEVD-FMK, which inhibits a cellular caspase in such a way that virus multiplication is inhibited,
**characterised in that**,
in addition, at least one antivirally acting substance, selected from the group consisting of the MEK inhibitors 2-(2-amino-3-methoxyphenyl)-4-oxo-4H-(1)benzopyran, U0126, PD18453, PD98059, is contained therein.

2. Use of at least one caspase inhibitor, selected from the group consisting of caspase 3 inhibitors, i.e. Z-DEVD-FMK, Ac-DEVD-CHO, Ac-DMQD-CHO, Z-D(OMe)E(OMe)VD(OMe)-FMK, Z-D(OMe)QMD(OMe)-FMK, and inhibitors of cellular caspases which are capable of activating caspase 3, i.e. the caspase 8 inhibitors Z-LE(OMe)TD(OMe)-FMK, Ac-ESMD-CHO, Ac-IETD-CHO, Z-IETD-FMK, the caspase 9 inhibitors Z-LE(OMe)HD(OMe)-FMK, Z-LEHD-FMK, Ac-LEHD-CHO, and the caspase 10 inhibitors Ac-AEVD-CHO, Z-AEVD-FMK, and an inhibitor of one or more cellular kinases, selected from the group consisting of the MEK inhibitors 2-(2-amino-3-methoxyphenyl)-4-oxo-4H-(1)benzopyran, U0126, PD18453, PD98059 for the preparation of a combination preparation according to claim 1 for the treatment of an influenza infection.

## Revendications

1. Préparation combinée pour son utilisation dans la thérapie d'une maladie virale provoquée par des virus influenza contenant au moins une substance active choisie dans le groupe constitué des inhibiteurs de la caspase 3, à savoir Z-DEVD-FMK, Ac-DEVD-CHO, Ac-DMQD-CHO, Z-D(OMe)E(OMe)VD(OMe)-FMK, Z-D(OMe)QMD(OMe)-FMK et des inhibiteurs des caspases cellulaires qui peuvent activer la caspase 3, à savoir les inhibiteurs de la caspase 8 Z-LE(OMe)TD(OMe)-FMK, Ac-ESMD-CHO, Ac-IETD-CHO, Z-IETD-FMK, les inhibiteurs de la caspase 9 Z-LE(OMe)HD(OMe)-FMK, Z-LEHD-FMK, Ac-LEHD-CHO, et les inhibiteurs de la caspase 10 Ac-AEVD-CHO, Z-AEVD-FMK, qui inhibe une caspase cellulaire de manière à inhiber une multiplication virale,
**caractérisée en ce qu'**elle contient en outre au moins une substance ayant une action antivirale, choisie dans le groupe constitué des inhibiteurs de MEK 2-(2-amino-3-méthoxyphényl)-4-oxo-4H-(1) benzopyrane, U0126, PD18453, PD98059.

2. Utilisation d'au moins un inhibiteur de caspase choisi dans le groupe constitué des inhibiteurs de la caspase 3, à savoir Z-DEVD-FMK, Ac-DEVD-CHO, Ac-DMQD-. CHO, Z-D(OMe)E(OMe)VD(OMe)-FMK, Z-D(OMe)QMD(OMe)-FMK et des inhibiteurs des caspases cellulaires qui peuvent activer la caspase 3, à savoir les inhibiteurs de la caspase 8 Z-LE(OMe)TD(OMe)-FMK, Ac-ESMD-CHO, Ac-IETD-CHO, Z-IETD-FMK, les inhibiteurs de la caspase 9 Z-LE(OMe)HD(OMe)-FMK, Z-LEHD-FMK, Ac-LEHD-CHO et les inhibiteurs de la caspase 10 Ac-AEVD-CHO, Z-AEVD-FMK, et un inhibiteur d'une ou plusieurs kinases cellulaires, choisi dans le groupe constitué des inhibiteurs de MEK 2-(2-amino-3-méthoxyphényl)-4-oxo-4H-(1) benzopyrane, U0126, PD18453, PD98059, pour la fabrication d'une préparation combinée selon la revendication 1 pour le traitement d'une infection à influenza.
